# EUROPEAN PATENT SPECIFICATION

(11) **EP 2 411 338 B1**
(45) Date of publication and mention of the grant of the patent: **16.12.2015**
(21) Application number: 09798925.5
(22) Date of filing: 21.12.2009
(51) Int. Cl.: C02F 3/28, C02F 11/04, C02F 11/12, C02F 11/14, C02F 11/16

(54) **BIOGAS PRODUCING SYSTEM**
SYSTEM ZUR HERSTELLUNG VON BIOGAS
SYSTÈME DE PRODUCTION D'UN BIOGAZ

(30) Priority: 25.03.2009 SE 0900376
(43) Date of publication of application: 01.02.2012
(73) Proprietor: Scandinavian Biogas Fuels AB, 111 22 Stockholm (SE)
(72) Inventor: EJLERTSSON, Jörgen, S-590 46 Rimforsa (SE)
(74) Representative: Sjölander, Henrik
(86) International application number: PCT/EP2009/067643
(87) International publication number: WO 2010/108558

(56) References cited:
- WO-A2-2009/118304
- GB-A- 2 003 459
- JP-A- 56 150 494
- DATABASE WPI Week 200347 Thomson Scientific, London, GB; AN 2003-497567 XP002578213 -& JP 2003 024975 A (DOKURITSU GYOSEI HOJIN SANGYO GIJUTSU SO) 28 January 2003 (2003-01-28)
- DAVIDSSON ASA ET AL: "PRE-TREATMENT OF WASTEWATER SLUDGE BEFORE ANAEROBIC DIGESTION-HYGIENISATION, ULTRASONIC TREATMENT AND ENZYME DOSING", VATTEN, FOERENINGEN FOER VATTENHYGIEN, STOCKHOLM, SW, vol. 62, 1 January 2006 (2006-01-01), pages 335-340, XP003025120, ISSN: 0042-2886

## Description

### Technical field

The present invention relates to a biogas producing system provided with means to reuse nutriments harvested from an earlier biogas producing system in a subsequent system to improve microorganism efficiency.

### Background

It is well known in the prior art that sludge from a process in a biogas producing system is dewatered and partially reused in the same process, as described in the published European patent application EP 1914205. Any nutriments, such as cobalt, selenium, tungsten, nickel, etc. present in the sludge may also be reused in the same process to avoid the need to introduce elementary substances as nutriments to the microorganisms in the biogas producing system.

A continuous supply of microorganisms needed to produce biogas may also be introduced into the tank reactor of the biogas producing system by mixing a part of the digested sludge with organic matter at the inlet, as described in the published international application WO 2007/114787, paragraphs [0023]-[0025].

Pre-treatment of organic matter before introducing it into a biogas producing system is also well known in the prior art, as disclosed in the article with the title " Pre-treatment of wastewater sludge before anaerobic digestion-hygienisation, ultrasonic treatment and enzyme dosing" by Åsa Davidsson and Jes La Cour Jansen, published in VATTEN 62:335-340, Lund 2006. Pre-treatment, e.g. hygienisation by thermal treatment, will increase the methane potential in biological sludge since the pre-treated organic matter is more accessible to the microorganisms in the tank reactor of the biogas producing system.

DE 102007005786 discloses a dryer in which the digested sludge from a biogas producing system is dewatered and pellets are created from the drying process. The pellets may thereafter be used as fertilizer.

Treatment of digested sludge before it is reused as nutriments in an anaerobic biogas producing process has also been proposed. The treatment described includes: thermal gasification for 10 minutes (US 4,289,625), hydrolyzing (US 5,141,646), and oxidation (US 5,492,624).

GB2003459 discloses a process for the utilization of anaerobic digested sewage sludge in which the sludge is heated and separated into a concentrate and a supernatant. A culture medium is prepared from the supernatant and is inoculated with anaerobic digested sewage sludge. The culture medium is anaerobic fermented to form biomass.

### Summary of the invention

An object with the present invention is to provide a method for producing biogas that requires less nutriments in the shape of chemical compounds of elementary substances to feed the microorganisms in a biogas reactor when producing biogas compared to the prior art.

The object is achieved by the method of claim 1 comprising feeding organic matter into a first tank reactor containing biogas producing microorganisms for digestion under anaerobic conditions in order to produce biogas. Digested sludge, having a desired composition of nutriments, from an anaerobic digestion process in a second tank reactor is provided and the nutriments are fed into the first tank reactor.

An advantage with the present invention is that digestion processes based on organic material with low concentration of necessary nutriments, e.g. whole stillage from an ethanol production plant, slaughter waste, crops, sugar beats, silage, starch, dairy waste, etc., may be greatly improved without having to add any nutriments in the form of chemical compounds of elementary substances as in prior art.

Another advantage and aspect of the present invention is that an increased amount of biogas may be produced in an anaerobic digestion process for producing biogas compared to prior art systems.

In a preferred embodiment the digested sludge is treated by hygienisation to form a nutriment additive, and the nutriment additive is fed to the biogas producing microorganisms in the first tank reactor.

An advantage with the preferred embodiment is that nutriments contained in the hygienised sludge from one biogas producing facility may be more easily transported to another remote located biogas producing facility compared to prior art systems.

Further objects and advantages will be apparent for a skilled person from the detailed description and the accompanying drawings.

### Brief description of the drawings

Figure 1 shows a prior art system for producing biogas from organic matter with dewatering of the digested sludge.
Figure 2 shows a prior art system for producing biogas from organic matter with pre-treatment of organic matter.
Figure 3 shows a first embodiment of a system for producing biogas from organic matter.
Figure 4 shows a second embodiment of a system for producing biogas from organic matter.

### Detailed description of preferred embodiments

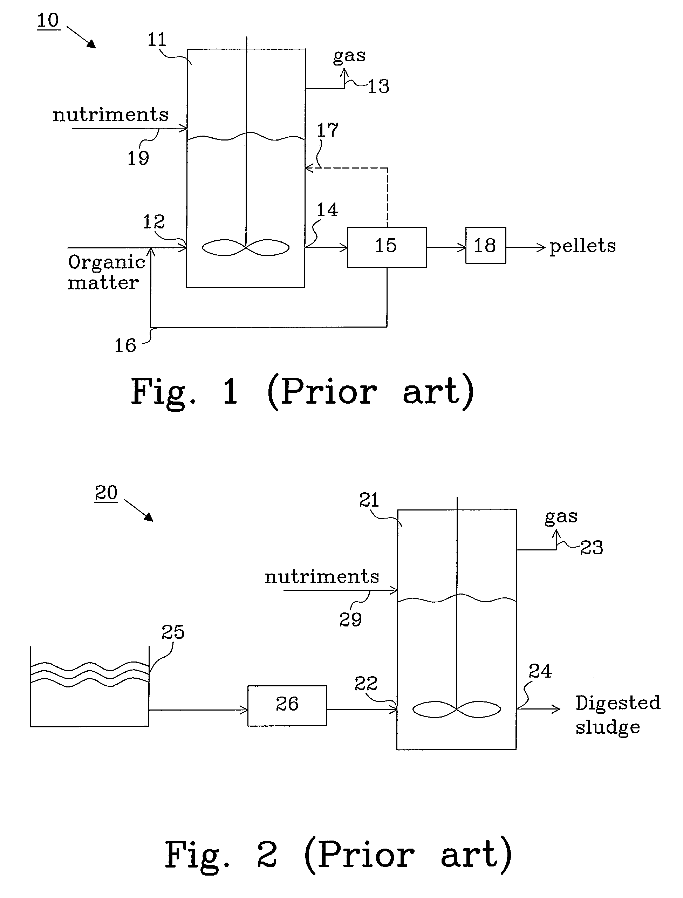

Figure 1 shows a first prior art system 10 for producing biogas from organic matter introduced into a tank reactor 11 via a tank inlet 12. The introduced organic matter is brought into contact with biogas producing microorganisms for digestion under anaerobic condition, and the organic matter is digested while producing biogas, as indicated by reference numeral 13.

Digested sludge is also produced as a result of the anaerobic digestion process in the tank reactor 11, which is available at a tank outlet 14. The digested sludge is dewatered in a dewatering device 15 producing dewatered sludge and reject water.

The reject water may be added to the organic matter introduced via the tank inlet 12, as indicated by the optional feeding pipe 16, and the digested sludge is suitable as a fertilizer but may also be reused in the anaerobic digestion process in the tank reactor as indicated by the optional feedback pipe 17. It is also possible to further treat the digested sludge by drying it in a dryer 18 to form the fertilizer into the shape of pellets.

The biogas producing microorganisms within the tank reactor continuously needs to be provided with nutriments, i.e. chemical compounds of elementary substances, to function properly, as indicated by reference numeral 19.

Figure 2 shows a second prior art system 20 for producing biogas from organic matter introduced into a tank reactor 21 via a tank inlet 22. The introduced organic matter is brought into contact with biogas producing microorganisms for digestion under anaerobic condition, and the organic matter is digested while producing biogas, as indicated by reference numeral 23. Digested sludge is also produced as a result of the anaerobic digestion process in the tank reactor 21, which is available at a tank outlet 24.

The organic matter originates from a waste water treatment plant 25 and the waste water is subjected to a pre-treatment process (e.g. ultrasound treatment, hygienisation, enzyme dosing, etc.) in a pre-treatment unit 26 before it is introduced into the tank reactor 21 via the tank inlet 22. Nutriments, i.e. chemical compounds of elementary substances, also need to be added in order to provide a suitable environment for the biogas producing microorganisms, as indicated by reference numeral 29.

The prior art systems described in connection with figures 1 and 2 disclose treatment of the organic matter before digestion and treatment of the digested sludge after digestion. The purpose of the prior art system is to increase the biogas potential of the organic matter as such, but both systems require adding nutriments in the shape of chemical compounds of elementary substances to the biogas producing microorganisms to obtain a suitable biogas producing environment within the tank reactors 11 and 21.

Nutriments in the shape of chemical compounds of elementary substances, such as cobalt (Co), selenium (Se), tungsten (W) and nickel (Ni), are expensive and restrictions to use these substances are currently discussed within the European Union.

One fundamental purpose of the present invention is to harvest the nutriments available in digested sludge from an anaerobic process; expose the digested sludge to a hygienisation process to kill off pathogens and optionally to reduce the volume of the digested sludge in a dewatering process; and to reuse the harvested nutriments in the same, or different, anaerobic process to reduce the need to add nutriments in the shape of chemical compounds of elementary substances (i.e. elementary substances, or elementary substances in its ionic form).

The environment within the tank reactor primarily depends on the type of organic matter that is introduced into the tank reactor, and in order to create a suitable biogas producing environment nutriments need to be added for the microorganisms to function properly. Examples of elementary substances used in chemical compounds as nutriments are: nitrogen (N), phosphorus (P), calcium (Ca), sulphur (S), iron (Fe), cobalt (Co), selenium (Se), tungsten (W) and nickel (Ni). When certain types of organic matter are used, such as sugar beats, it is also essential to introduce nutriments that have an alkaline increasing effect, i.e. increases the pH in the tank reactor.

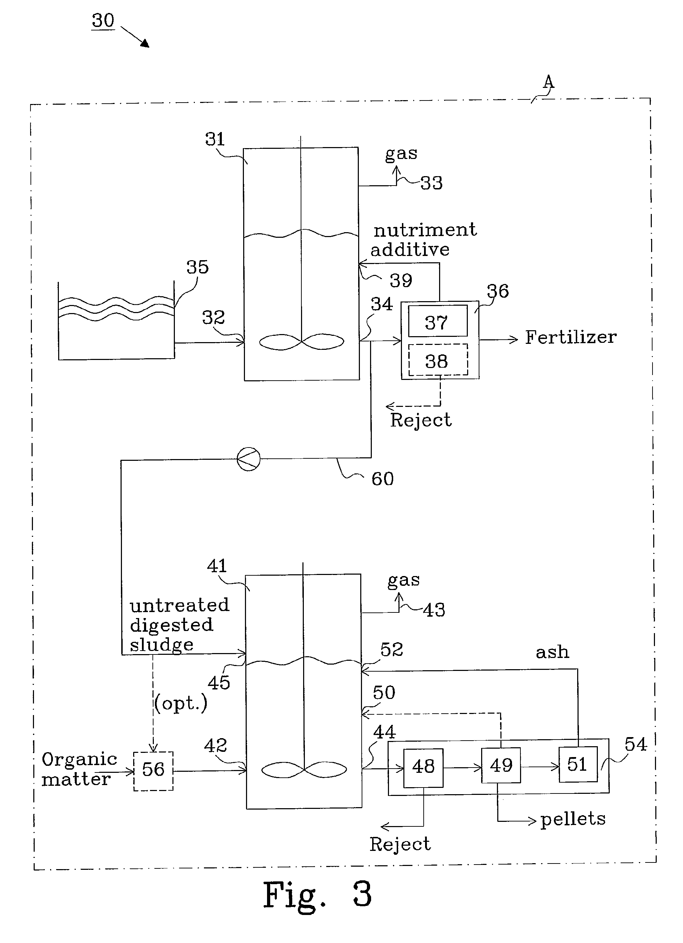

Figure 3 shows a first embodiment of a system 30 for producing biogas from organic matter. The system comprises a first tank reactor 41 and a second tank reactor 31, which in this embodiment are arranged at the same location "A" (as indicated by dash dotted lines). The organic matter is introduced into the second tank reactor 31 via a tank inlet 32. The introduced organic matter is brought into contact with biogas producing microorganisms for digestion under anaerobic condition, and the organic matter is digested while producing biogas, as indicated by reference numeral 33. Digested sludge is also produced as a result of the anaerobic digestion process in the second tank reactor 31, which is available at a tank outlet 34.

The organic matter introduced into the second tank reactor 31 originates in this embodiment from a waste water treatment plant 35, and is introduced into the second tank reactor without any pre-treatment. It is naturally possible to include a pre-treatment device as described in connection with figure 2 to further enhance the biogas potential in the organic matter.

A treatment unit 36 receives digested sludge from the tank outlet 34, and transforms the digested sludge into a nutriment additive. The treatment unit 36 includes a hygienisation device 37 and optionally a dewatering device 38 (as indicated by dashed lines). In addition to hygienisation of the digested sludge, the hygienisation device 37 may also reduce the amount of water in the digested sludge and stabilise the digested sludge depending on the chosen hygienisation process (as described below).

Organic matter introduced into the first tank reactor 41 via a feeder attached to a tank inlet 42. The introduced organic matter is brought into contact with biogas producing microorganisms for digestion under anaerobic condition, and the organic matter is digested while producing biogas, as indicated by reference numeral 43. Digested sludge is also produced as a result of the anaerobic digestion process in the first tank reactor 41, which is available at a tank outlet 44.

A treatment unit 54 receives digested sludge from the tank outlet 44, and transforms the digested sludge into a nutriment additive. The treatment unit 54 includes in this embodiment a dewatering device 48 producing reject water that may be reused in the process, a hygienisation device 49 and a furnace 51. The hygienisation device 49 is in this embodiment realised by a dryer or a chemical dispensing unit configured to add lime/slaked lime, since the dewatering process is performed prior to the hygienisation. The nutriment additive from the hygienisation may be introduced (dashed line) into the first tank reactor 41 via a first nutriment inlet 50, and/or available as pellets for other applications, such as fertilizer.

A part of the dried sludge (or nutriment additive) from the hygienisation device 49 is forwarded to the furnace 51 to produce a nutriment rich ash introduced via a second nutriment inlet 52. It should be noted that the nutriment rich ash also has a pH increasing effect on the organic matter within the first tank reactor, which may be essential if the organic matter introduced at the tank inlet 42 has a low pH. Preferably, a combination of nutriment rich ash and nutriment additive is introduced into the first tank reactor 41, e.g. 70 % nutriment rich ash and 30 % nutriment additive, to obtain a suitable pH environment and reuse of nutriments present in the digested sludge.

The organic matter introduced into the tank inlet 42 has, in this embodiment, a low concentration of nutriments and low level of protein, such as silage, sugar beats, starch, cocking oil, vegetable based oil, fibres from pulp industry, effluent from cellulose based ethanol production (i.e. cellulose and hemicellulose), etc. Nutriments, as defined above, are needed in order to create a suitable environment for the biogas producing microorganisms is provided through a separate nutriment inlet 45. Nutriments are in this embodiment provided as untreated digested sludge from the outlet 34 of the second tank reactor 31.

### Hygienisation processes

A first example of a hygienisation process is drying the digested sludge in a dryer. rying at a temperature of 70 degrees Celsius for one hour will be sufficient to hygienisate the digested sludge as well as reduce the amount of water and stabilise the digested sludge.

A second example of a hygienisation process is a chemical process controlled by a chemical dispensing unit configured to add hydrogen peroxide H₂O₂ into the digested sludge. The hydrogen peroxide will hygienisate the digested sludge during a chemical oxidation reaction. The oxidation reaction will also improve the possibility to reduce the amount of water in the hygienisated digested sludge during a subsequent dewatering procedure, see below, which stabilises the digested sludge, and reduces the amount of water.

A third example of a hygienisation process is another chemical process controlled by the chemical dispensing unit configured to add lime CaO and/or slaked lime Ca(OH)₂ into the digested sludge. The lime/slaked lime will hygienisate the digested sludge by generating a high pH level that kill any pathogens within the digested sludge. A dewatering process will stabilise the digested sludge, as mentioned below.

In order to further reduce the amount of water in the nutriment additive, i.e. the treated digested sludge, a dewatering device 38 is preferably implemented in the treatment unit 36. If the hygienisation device 37 is implemented as a dryer (as described above), the digested sludge should be dewatered in the dewatering device 38 prior to hygienisation to achieve the best result. On the other hand if the hygienisation device 37 is a chemical dispensing unit configured to add hydrogen peroxide, the dewatering process should be performed after hygienisation, since the addition of hydrogen peroxide will help the dewatering device to produce cleaner reject water due to the binding of substances in the digested sludge.

However, if the hygienisation device 37 is a chemical dispensing unit configured to add lime/slaked lime, the dewatering process may be performed prior to or after the hygienisation.

Organic matter, such as grass, may be mixed with a percentage of digested sludge before hygienisation when the hygienisation process is performed by drying, and pellets may be formed containing a mixture of organic matter and necessary nutriments in a suitable ratio, e.g. 90% grass and 10% digested sludge. The pellets may thereafter be fed to a subsequent biogas producing process without having to add additional organic matter or any nutriments in the form of elementary substances.

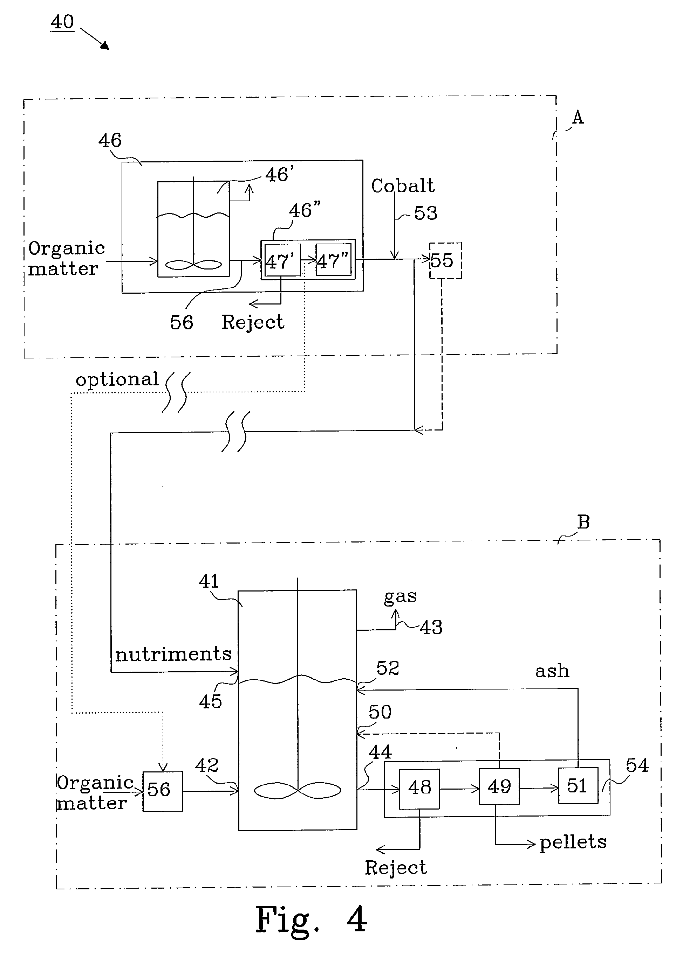

Figure 4 shows a second embodiment of a system 40 for producing biogas from organic matter comprising a first tank reactor 41 arranged at a first location "A" and a second tank reactor 46 arranged at a second location "B". The first tank reactor 41 has been described in connection with figure 3, but the nutriments provided to the nutriment inlet 45 are transported from the second location "B" Nutriments are in this embodiment provided in the shape of pellets, such as the nutriment additives obtainable from the treatment device 36 in figure 3, or dried sludge from a previous anaerobic process such as the biogas producing system including the second tank reactor 46, similar to the system described in connection with figure 1, also comprising a treatment unit 46" including a dewatering device 47' and a hygienisation device 47". Optionally, cobalt may be added to the pellets as indicated by 53, and the pellets are transported to the separate nutriment inlet 45 of the first tank reactor 41. In a preferred embodiment, the pellets (with or without the added cobalt) is further treated in a furnace 55 before transported to the nutriment inlet 45.

Normally the distance between the first tank reactor 41 and the second tank reactor is great and the cost for transporting the nutriment additive/nutriment rich ash depends on the weight and volume. The treatment unit 46" will reduce the weight by 96-98 %, i.e. 100 tons of digested sludge at 2-4 % TS and 60 % VS will be reduced to 2-4 tons of nutriment additive (dried sludge) at 70-90 % TS and 65 % VS. In order to further reduce the weight, the optional furnace 55 may be included in the treatment unit 46", preferably at the site of the second tank reactor 46', and the furnace 55 will further reduce the weight of the dried sludge by approximately 65 %, i.e. 100 tons of digested sludge at 2-4 % TS and 60 % VS will be reduced to approximately 700-1400 kg of nutriment rich ash.

The two embodiments described in connection with figures 3 and 4 illustrate the inventive concept of reusing the nutriments available in the digested sludge from an earlier biogas producing tank reactor in a subsequent biogas producing tank reactor. The transport of the digested sludge to the subsequent biogas producing tank reactor may be accomplished by pumping the digested sludge through a pipeline (if the distance between the tank reactors are not too great) or by surface transport (trucks, railroad, etc.) if the distance is large. When pumping the digested sludge through a pipeline, it is preferably that the digested sludge is untreated in order to be pumpable, but when using surface transport, the weight and volume are of more importance which require some kind of treatment as illustrated above.

It should be noted that other types of transport between tank reactors situated at the same site may be implemented in any suitable way to facilitate transport of untreated, or treated, digested sludge.

## Claims

1. A method for producing biogas by anaerobic digestion of organic matter, said method comprising:
a) feeding organic matter suitable for biogas production to a first tank reactor (41), and in said first tank reactor (41), contacting said organic matter with biogas producing microorganisms for digestion under anaerobic conditions, and
b) digesting said organic matter in said first tank reactor (41) while producing biogas,
c) providing digested sludge from an anaerobic digestion process in a second tank reactor (31; 46), different than said first tank reactor (41), said digested sludge containing a desired composition of nutriments, and
d) feeding said nutriments into said first tank reactor (41)
**characterized in that** said method further comprises:
c1) performing a first treatment of said digested sludge after step c), said first treatment comprises:
c1.1) hygienisating said digested sludge in a hygienisation device (47") to form a nutriment additive; and
c1.2) dewatering said digested sludge in a dewatering device (4V') either prior to step c1.1) to form a dewatered digested sludge to be hygienisated in step c1.1) to form a nutriment additive or after step c1.1) to form a dewatered nutriment additive;
before feeding said dewatered nutriment additive to said biogas producing microorganisms in said first tank reactor (41) in step d).

2. The method according to claim 1, wherein step c1.1) is performed by drying said digested sludge to form said nutriment additive.

3. The method according to claim 1, wherein step c1.1) is performed by adding hydrogen peroxide to said digested sludge to form said nutriment additive.

4. The method according to claim 1, wherein step c1.1) is performed by adding lime CaO and/or slaked lime Ca(OH)₂ to said digested sludge to form said nutriment additive.

5. The method according to any of claims 1-4, wherein said method further comprises:
c2) performing a second treatment to burn said nutriment additive in a furnace (55) after step c1) to produce a nutriment rich ash before feeding at least a part of said nutriment rich ash to said biogas producing microorganisms in said first tank reactor (41) in step d).

## Patentansprüche

1. Verfahren zur Produktion von Biogas durch anaeroben Aufschluss organischer Materie, wobei das Verfahren Folgendes umfasst:
a) Einspeisen organischer Materie, die für Biogas-Produktion geeignet ist, in einen ersten Reaktortank (41) und in dem ersten Reaktortank (41) Inkontaktbringen der organischen Materie mit Biogas-produzierenden Mikroorganismen zum Aufschluss unter anaeroben Bedingungen, und
b) Aufschließen der organischen Materie in dem ersten Reaktortank (41) unter Produktion von Biogas,
c) Bereitstellen von aufgeschlossenem Schlamm eines anaeroben Aufschlussprozesses in einem zweiten Reaktortank (31; 46), der sich von dem ersten Reaktortank (41) unterscheidet, wobei der aufgeschlossene Schlamm eine erwünschte Zusammensetzung von Nährstoffen enthält, und
d) Einspeisen der Nährstoffe in den ersten Reaktortank (41), **dadurch gekennzeichnet, dass** das Verfahren weiterhin Folgendes umfasst:
c1) Durchführen einer ersten Behandlung des aufgeschlossenen Schlamms nach Schritt c), wobei die erste Behandlung Folgendes umfasst:
c1.1) Hygienisierung des aufgeschlossenen Schlamms in einer Hygienisierungsvorrichtung (47"), um ein Nährstoffadditiv zu bilden; und
c1.2) Entwässern des aufgeschlossenen Schlamms in einer Entwässerungsvorrichtung (47') entweder vor Schritt c1.1), um einen entwässerten aufgeschlossenen Schlamm zur Hygienisierung in Schritt c1.1) zu bilden,
um ein Nährstoffadditiv zu bilden, oder nach Schritt c1.1), um ein entwässertes Nährstoffadditiv zu bilden;
vor Einspeisen des entwässerten Nährstoffadditivs zu den Biogas-produzierenden Mikroorganismen in dem ersten Reaktortank (41) in Schritt d).

2. Verfahren nach Anspruch 1, wobei Schritt c1.1) durch Trocknen des aufgeschlossenen Schlamms ausgeführt wird, um das Nährstoffadditiv zu bilden.

3. Verfahren nach Anspruch 1, wobei Schritt c1.1) durch Zugabe von Wasserstoffperoxid zu dem aufgeschlossenen Schlamm ausgeführt wird, um das Nährstoffadditiv zu bilden.

4. Verfahren nach Anspruch 1, wobei Schritt c1.1) durch Zugabe von Branntkalk CaO und/oder Löschkalk Ca(OH)₂ zu dem aufgeschlossenen Schlamm ausgeführt wird, um das Nährstoffadditiv zu bilden.

5. Verfahren nach einem der Ansprüche 1 bis 4, wobei das Verfahren weiterhin Folgendes umfasst:
c2) Durchführen einer zweiten Behandlung, um das Nährstoffadditiv nach Schritt c1) in einem Ofen (55) zu verbrennen, um eine nährstoffreiche Asche zu produzieren, bevor mindestens ein Teil der nährstoffreichen Asche zu den Biogas-produzierenden Mikroorganismen in dem ersten Reaktortank (41) in Schritt d) eingespeist wird.

## Revendications

1. Procédé de production d'un biogaz par digestion anaérobie de matière organique, ledit procédé comprenant :
a) l'alimentation par ladite matière organique appropriée à la production de biogaz d'un premier réacteur de type cuve (41), et dans ledit réacteur de type cuve (41), la mise en contact de ladite matière organique avec des microorganismes produisant un biogaz pour la digestion en conditions anaérobies, et
b) la digestion de ladite matière organique dans ledit premier réacteur de type cuve (41) en produisant un biogaz,
c) l'obtention d'une boue digérée par un processus de digestion anaérobie dans un second réacteur de type cuve (31 ; 46), différent dudit premier réacteur de type cuve (41), ladite boue digérée contenant une composition de nutriments désirée, et
d) l'alimentation par lesdits nutriments dudit premier réacteur de type cuve (41),
**caractérisé en ce que** ledit procédé comprend en outre :
c1) l'application d'un premier traitement sur ladite boue digérée après l'étape c), ledit premier traitement comprenant :
c1.1) l'hygiénisation de ladite boue digérée dans un dispositif d'hygiénisation (47") pour former un additif de nutriments ; et
c1.2) la déshydratation de ladite boue digérée dans un dispositif de déshydratation (47'), soit avant l'étape c1.1) pour former une boue digérée déshydratée à hygiéniser dans l'étape c1.1) pour former un additif de nutriments, soit après l'étape c1.1) pour former un additif de nutriments déshydraté ;
avant d'alimenter, par ledit additif de nutriments déshydraté, lesdits microorganismes produisant un biogaz dans ledit réacteur de type cuve (41) dans l'étape d).

2. Procédé selon la revendication 1, dans lequel l'étape c1.1) est mise en oeuvre par séchage de ladite boue digérée pour former ledit additif de nutriments.

3. Procédé selon la revendication 1, dans lequel l'étape c1.1) est mise en oeuvre par addition de peroxyde d'hydrogène à ladite boue digérée pour former ledit additif de nutriments.

4. Procédé selon la revendication 1, dans lequel l'étape c1.1) est mise en oeuvre par addition de chaux CaO et/ou de chaux éteinte Ca(OH)₂ à ladite boue digérée pour former ledit additif de nutriments.

5. Procédé selon l'une quelconque des revendications 1 à 4, ledit procédé comprenant en outre :
c2) l'application d'un second traitement pour brûler ledit additif de nutriments dans un four (55) après l'étape c1) afin de produire une cendre riche en nutriments avant d'alimenter, par au moins une partie de ladite cendre riche en nutriments, lesdits microorganismes produisant un biogaz dans ledit premier réacteur de type cuve (41) à l'étape d).
